# EUROPEAN PATENT SPECIFICATION

(11) **EP 2 969 020 B1**
(45) Date of publication and mention of the grant of the patent: **29.11.2017**
(21) Application number: 14714085.9
(22) Date of filing: 10.03.2014
(51) Int. Cl.: C07C 33/02, C07C 33/025, C07C 69/07, C07C 309/20, C07C 309/29, C07C 15/44, C11D 1/14, C11D 1/22, C11D 3/18, C11D 3/20, C07C 67/283, C07C 67/293, C07C 67/475, C07C 69/007, C11D 1/00

(54) **SPECIFIC UNSATURATED AND BRANCHED FUNCTIONAL MATERIALS FOR USE IN CONSUMER PRODUCTS**
SPEZIELLE UNGESÄTTIGTE UND VERZWEIGTE FUNKTIONSMATERIALIEN ZUR VERWENDUNG IN VERBRAUCHERPRODUKTEN
MATÉRIAUX FONCTIONNELS NON SATURÉS ET RAMIFIÉS SPÉCIFIQUES POUR LEUR UTILISATION DANS DES PRODUITS DE CONSOMMATION

(30) Priority: 15.03.2013 US 201361792510 P
(43) Date of publication of application: 20.01.2016
(73) Proprietor: The Procter & Gamble Company, Cincinnati, OH 45202 (US)
(72) Inventor: SCHEIBEL, Jeffrey, John, Cincinnati, Ohio 45202 (US); WEST, Ryan, Michael, Cincinnati, Ohio 45202 (US); CRON, Scott, Leroy, Cincinnati, Ohio 45202 (US); DEROSE, Stephen, Anthony, Cincinnati, Ohio 45202 (US); LINGOES, Janette, Villalobos, Cincinnati, Ohio 45202 (US); KELLETT, Patti, Jean, Cincinnati, Ohio 45202 (US); URBIN, Stephanie, Ann, Cincinnati, Ohio 45202 (US)
(74) Representative: Peet, Jillian Wendy
(86) International application number: PCT/US2014/022481
(87) International publication number: WO 2014/150171

(56) References cited:
- EP-A1- 0 068 506
- EP-A2- 0 255 904
- EP-A2- 0 409 181
- WO-A2-2010/033976
- CH-A- 473 889
- DD-A- 13 617
- DE-A1- 2 307 468
- US-A- 3 917 713
- US-A1- 2013 085 283
- US-B1- 6 586 461
- RAWAT D S ET AL: "Synthesis of 7-Substituted Farnesyl Diphosphate Analogues", ORGANIC LETTERS, AMERICAN CHEMICAL SOCIETY, US, vol. 4, no. 18, 2002, pages 3027-3030, XP003013356, ISSN: 1523-7060, DOI: 10.1021/OL026176I
- R. BADET ET AL: "C-Prenylation of Isopentyl Derivatives with Sulfonium Salts", TETRAHEDRON LETTERS, vol. 26, no. 16, 1985, pages 2007-2010, XP002726427,
- GRÜNANGER ET AL: "Branched-Regioselective Hydroformylation with Catalytic Amounts of a Reversibly Bound Directing Group. Supporting Information", ANGEWANDTE CHEMIE INTERNATIONAL EDITION, vol. 47, no. 38, 8 September 2008 (2008-09-08), pages 7346-7349, XP55026702, ISSN: 1433-7851, DOI: 10.1002/anie.200802296
- BAHMAN TAMAMI ET AL: "Polyvinylpyridine Chloroaluminium Borohydride As a New Stable, and Efficient Reducing Agent in Organic Synthesis", IRANIAN POLYMER JOOURNAL, vol. 6, no. 3, 1997, pages 159-167, XP002726428,
- GOSSELIN P ET AL: "Stereospecific synthesis of homogeranic and homoneric acids", SYNTHESIS, GEORG THIEME VERLAG, STUTTGART, DE, vol. 10, 1984, pages 876-881, XP002218337, ISSN: 0039-7881, DOI: 10.1055/S-1984-31007
- ROBERT J. ELY AND JAMES P. MORKEN: "Regio- and Stereoselective Ni-Catalyzed 1,4-Hydroboration of 1,3-Dienes: Access to Stereodefined (Z)-Allylboron Reagents and Derived Allylic Alcohols", JACS COMMUNICATIONS, vol. 132, 2 May 2010 (2010-05-02), pages 2534-2535, XP002726429,
- DANA P. SIMMONS ET AL: "Aldehyde Enol Esters as Novel Chain Terminators in Cationic Olefin Cyclizations", HELV. CHIM. ACTA, vol. 71, 1988, pages 1000-1004, XP002726430,
- ARCHANA RANI ET AL: "Diels-Alder Reaction of In-situ Generated p-Benzoquinones with Isoprenoidal Dienol Acetate: Novel Synthesis of 2-Isoprenoidal Naphthalene-5,8-Quinols", TETRAHEDRON LETTERS, vol. 37, no. 44, 1996, pages 8037-8040, XP002726431,
- TIM DEN HARTOG ET AL: "Catalytic Enantioselective 1,6-Conjugate Addition of Grignard Reagents to Linear Dienoates", ANGEW. CHEM. INT. ED., vol. 47, 2008, pages 398-401, XP002726432,
- ALEXANDER KÖPFER ET AL: "Regiodivergent reductive coupling of 2-substituted dienes to formaldehyde employing ruthenium or nickel catalyst: hydrohydroxymethylation via transfer hydrogenation", CHEM. SCI., vol. 4, 11 February 2013 (2013-02-11), pages 1876-1880, XP002726433,
- CHRISTIAN T. GORALSKI AND DENNIS L. HASHA: "Hydroboration. 86. Convenient Conversion of Aldehydes and Ketones into the Corresponding Alkanes via Hydroboration of Their Enamines. A Remarkably Simple Synthesis of Either (Z)- or (E)-Alkenes", J. ORG. CHEM., vol. 56, 1991, pages 1543-1549, XP002726434,
- BARNES R A ET AL: "CHEMICAL TRANSFORMATIONS OF CITRONELLAL", INTERNATIONAL CONGRESS OF ESSENTIAL OILS, XX, XX, vol. 7, 1977, pages 253-256, XP001078886,
- ALEXANDER ST. PFAU AND PL. PLATTNER: "Reduktion der Glycid-ester zu 1,3-Glykolen und deren Überführung in ungesättigte primäre Alkohole", HELV. CHIM. ACTA, vol. 15, no. 1, 1932, pages 1250-1267, XP002726435,
- B. BADET ET AL: "On the Regioselectivity of Elimination Reactions in Terpene Derivatives", TETRAHEDRON, vol. 44, no. 10, 1988, pages 2913-2924, XP002726436,
- DATABASE REAXYS [Online] Elsevier Properties SA; XP002726437, Database accession no. 1854292 & JOURNAL OF THE AMERICAN CHEMICAL SOCIETY, vol. 94, 1972, pages 2286-2290,
- DATABASE REAXYS [Online] Elsevier Properties SA; XP002726438, Database accession no. 9387267 & J. AM. CHEM. SOC., vol. 125, no. 16, 2003,
- DATABASE REAXYS [Online] Elsevier Properties SA; XP002726439, Database accession no. 8541719 & ORGANIC LETTERS, vol. 2, no. 8, 2000, pages 1129-1131,
- DATABASE REAXYS [Online] Elsevier Properties SA; XP002726440, Database accession no. 4656881 & J. ORG. CHEM., vol. 48, no. 12, 1983, pages 2084-2090,
- DATABASE REAXYS [Online] Elsevier Properties SA; XP002726441, Database accession no. 2324246 & JOURNAL OF NATURAL PRODUCTS, vol. 66, no. 6, 2003, pages 743-751,
- DATABASE REAXYS [Online] Elsevier Properties SA; XP002726442, Database accession no. 1904831 & YUKI GOSEI KYOKAISHI, vol. 32, 1974, page 933,
- DATABASE REAXYS [Online] Elsevier Properties SA; XP002726443, Database accession no. 11200777 & ANGEWANDTE CHEMIE - INTERNATIONAL EDITION, vol. 46, no. 15, 2007, pages 2619-2622,
- DATABASE REAXYS [Online] Elsevier Properties SA; XP002726444, Database accession no. 4956277 & TETRAHEDRON LETTERS, vol. 29, no. 27, 1988, pages 3365-3368,
- DATABASE REAXYS [Online] Elsevier Properties SA; XP002726445, Database accession no. 22956552 & J. AM. CHEM. SOC., vol. 134, no. 36, 2012, pages 14760-14763,
- DATABASE REAXYS [Online] Elsevier Properties SA; XP002726446, Database accession no. 1757545 & TETRAHEDRON, vol. 44, no. 36, 2003, pages 6959-6961,
- DATABASE REAXYS [Online] Elsevier Properties SA; XP002726447, Database accession no. 1748060 & J. ORG. CHEM., vol. 41, 1976, pages 278-281,
- DATABASE REAXYS [Online] Elsevier Properties SA; XP002726449, Database accession no. 1904832 & CHEMISCHE BERICHTE, vol. 104, 1971, pages 1800-1806,
- DATABASE REAXYS [Online] Elsevier Properties SA; XP002726450, Database accession no. 6920617 & CHEMISTRY LETTERS, vol. 4, 1994, pages 767-770,
- DATABASE REAXYS [Online] Elsevier Properties SA; XP002726451, Database accession no. 23190868 & J. AM. CHEM. SOC., vol. 134, no. 51, 2012, pages 20844-20848,
- DATABASE REAXYS [Online] Elsevier Properties SA; XP002726452, Database accession no. 6967712 & PHYTOCHEMISTRY (ELSEVIER), vol. 23, no. 8, 1984, pages 1800-1802,
- DATABASE REAXYS [Online] Elsevier Properties SA; XP002726453, Database accession no. 4661729 & TETRAHEDRON LETTERS, vol. 44, no. 36, 2003, pages 6959-6961,
- DATABASE REAXYS [Online] Elsevier Properties SA; XP002726454, Database accession no. 8831125 & JOURNAL OF CHEMICAL ECOLOGY, vol. 27, no. 5, 2001, pages 995-1009,
- DATABASE REAXYS [Online] Elsevier Properties SA; XP002726455, Database accession no. 7905614 & HELV. CHIM. ACTA, vol. 83, no. 8, 2000, pages 2007-2022,
- P. KARRER AND M. STÄHELIN: "Über zwei neue Homologe des alpha-Tocopherols", HELV. CHIM. ACTA, vol. 24, no. 1, 1945, pages 438-443, XP002726456,
- DATABASE REAXYS [Online] Elsevier Properties SA; XP002726457, Database accession no. 9076946 & BIOORGANIC AND MEDICINAL CHEMISTRY LETTERS, vol. 11, no. 16, 2001, pages 2157-2159,
- KULKARNI S N ET AL: "Synthesis of PhytolIsomers: 7,11,15-tri-methyl-3-methylenehexadecanol & 3,7,11,15-tetramethylhexadec-3-en-1-ol & their conversion into vitamin E", INDIAN JOURNAL OF CHEMISTRY. SECTION B, COUNCIL OF SCIENTIFIC AND INDUSTRIAL RESEARCH (C S I R), IN, vol. 27, 1988, pages 65-66, XP009111717, ISSN: 0019-5103
- YAMAMURA Y ET AL: "BIOMIMETIC ENTRY TO ACYCLIC TERPENE SYNTHESIS A NOVEL REARRANGEMENT OF ALLYL ETHER CATALYZED BY ORGANOALUMINUM REAGENTS", TETRAHEDRON LETTERS, PERGAMON, GB, vol. 23, no. 18, 1982, pages 1933-1936, XP002912957, ISSN: 0040-4039, DOI: 10.1016/S0040-4039(00)87225-1
- ANGUS L. DAWE ET AL: "Novel Modifications to the Farnesyl Moiety of the a-Factor Lipopeptide Pheromone from Saccharomyces cerrevisiae: A role for Isoprene Modifications in Ligand Presentation", BIOCHEMISTRY, [Online] vol. 36, 1997, pages 12036-12044, XP002726458,
- A. M. MOISEENKOV ET AL: "Synthesis and structure of tricyclic furanosesquiterpenoids related to Pallescensin A", RUSSIAN CHEMICAL BULLETIN, vol. 43, no. 1, 1994, pages 153-160, XP002726459,
- ANTHONY D. WRIGHT ET AL: "Tropical Marine Algae, VII. The Chemical Composition of Marine Algae from North Queensland Waters", JOOURNAL OF NATURAL PRODUCTS, vol. 53, no. 4, 1990, pages 845-861, XP002726460,
- PAUL V J ET AL: "Diterpenoid feeding deterrents from the pacific green alga Pseudochlorodesmis furcellata", PHYTOCHEMISTRY, PERGAMON PRESS, GB, vol. 27, no. 4, 1988, pages 1011-1014, XP026633796, ISSN: 0031-9422, DOI: 10.1016/0031-9422(88)80262-0 [retrieved on 1988-01-01]
- A. J. BLACKMAN AND R. J. WELLS: "Flexilin and Trifarin, Terpene 1,4-Diacetoxybuta-1,3-dienes from two Caulerpa Species (Chlorophyta).", TETRAHEDRON LETTERS, no. 33, 1978, pages 3063-3064, XP002726461,
- GAVIN A. WHITLOCK AND EERICK M. CARREIRA: "Enantioselective Synthesis of ent-Stelettamide via a Novel Dipolar Cycloaddition Reaction of (Trimethylsilyl)diazomethane", J. ORG. CHEM., vol. 62, 1997, pages 7916-7917, XP002726462,
- NAOYUKI NISHIKAWA ET AL: "Stabilizing Effect of Diphytanylphosphate on Dipalmitoylphosphatidylcholine Bilayer Membrane", CHEMISTRY LETTERS, 1994, pages 767-770, XP002726463,
- DATABASE REAXYS [Online] Elsevier Properties SA; XP002726464, Database accession no. 1861403 & COMPTES RENDUS HEBDOMADAIRES DES SEANCES DE L'ACADEMIE DES SCIENCES, vol. 252, 1961, pages 1380-1382,
- DATABASE REAXYS [Online] Elsevier Properties SA; XP002726465, Database accession no. 1932041 & AGRICULTURAL AND BIOLOGICAL CHEMISTRY, vol. 35, 1971, page 1116,
- DATABASE REAXYS [Online] Elsevier Properties SA; XP002726466, Database accession no. 5253409 & TETRAHEDRON, vol. 44, no. 9, 1988, page 2913,
- DATABASE REAXYS [Online] Elsevier Properties SA; XP002726467, Database accession no. 4994413 & TETRAHEDRON, vol. 42, no. 9, 1986, pages 2485-2490,
- DATABASE REAXYS [Online] Elsevier Properties SA; XP002726468, Database accession no. 6861567 & AGRICULTURAL AND BIOLOGICAL CHEMISTRY, vol. 46, no. 3, 1982, pages 819-820,
- DATABASE REAXYS [Online] Elsevier Properties SA; XP002726469, Database accession no. 5517120 & BULLETIN DE LA SOCIETE CHIMIQUE DE FRANCE, vol. 2, no. 11-12, 1980, pages 588-600,
- DATABASE REAXYS [Online] Elsevier Properties SA; XP002726470, Database accession no. 6697439 & TETRAHEDRON, vol. 32, no. 14, 1976, pages 1675-1680,

## Description

### FIELD OF THE INVENTION

The present invention relates to a mixture of certain novel unsaturated branched functional compositions that are made via isoprenoids and/or isoprenoid derivatives which come from either natural or synthetic sources. Also disclosed is their use or modification for use in consumer products such as laundry products, personal care products, dishcare products, shampoo products and hard surface cleaning products, and the like comprising the functional compositions or modified compositions.

### BACKGROUND OF THE INVENTION

Surfactants, even today, are the single most important cleaning ingredient in laundry and household cleaning products. Anionic surfactants, as a class, are the largest in terms of worldwide consumption and typically are used at levels as high as 30 to 40% of the detergent formulation. Other important surfactants used in consumer products include amine oxides, cationic surfactants, zwitterionic surfactants, soaps, and fabric softening cationic surfactants. These surfactants provide additional cleaning benefits above and beyond what is provided by anionic surfactants, as well as other benefits such as enhanced foaming, enhanced skin mildness, and fabric softening. WO2010/033976 describes a process for making a detergent alcohol mixture from poly-branched poly-olefins. Derivatives of the alcohol mixture are then used in surfactant compositions. A need still exists for enhanced cold water cleaning performance, enhanced performance in general, and process and rheology advantages. Furthermore, it is highly desired that such materials be readily biodegradable and substantially derived from biomaterials to make consumer products with a better sustainability profile. The novel surfactant intermediates and surfactants disclosed herein solve many of the above needs to be useful in formulation of consumer products such as personal care, laundry and cleaning products.

### SUMMARY OF THE INVENTION

According to the present invention mixtures comprising poly-branched mono-unsaturated or poly-unsaturated derivatives of certain acyclic isoprenoids are provided. These mixtures can either be used as intermediates for further conversion to various surfactants or used unmodified, depending on the particular co-metathesis agent used to prepare the compounds. Thus, some of the mixtures are surfactants; others, when unmodified are conditioning agents, and thus can be used as is in consumer products. These compositions have improved properties over classical linear surfactants and conditioning agents.

The mixtures according to the present invention comprise a first composition and a second composition, wherein said first composition is represented by the general formula:

H[CH₂CH₀₊ₐ (CH₃)CH₁₊ₐ CH₂]ₓCH₂CH_{0+b} (CH_{1+b}R²)(CH_{1+c} CH_{1+c})R¹

wherein x = 1-5; a = 1; b = 1; c = 0; R¹=(CH2)ᵣ-Q and R² = H, wherein:
i. Q = O(CH2CH2O)ₚH, OCOCH₃,
ii. p = 0; and
iii. r = 1;
and wherein said second composition is represented by the general formula:

H[CH₂CH₀₊ₐ (CH₃)CH₁₊ₐ CH₂]ₓCH₂CH_{0+b} (CH_{1+b} R²)(CH_{1+c}CH_{1+c})R¹

wherein x = 1-5; a = 1; b = 0; c = 1; R¹= H and R² = (CH2)ᵣQ wherein:
i. Q = O(CH2CH2O)ₚH, OCOCH₃,
ii. p = 0; and
iii. r = 1.

In one embodiment, the composition has a bio-based content of greater than 50%. In another embodiment, the composition has a bio-based content of essentially 100%. The polybranched mono-olefin or poly-olefin containing compositions are prepared and/or modified by the following process (Process 1):
a. Co-metathesizing an isoprenoid and a second compound with a metathesis catalyst neat or in a solvent, wherein
   i. the isoprenoid has a structure of formula:

      H[CH₂CH₀₊ₐ (CH₃)CH₁₊ₐ CH₂]ₓCH₂CH_{0+b} (CH_{2+b})(CH_{1+c}CH_{2+c});

      and a = 0 or 1, b= 0 or 1, c= 0 or 1 and b+c = 0 or 1;
   ii. the second compound has structure of either formula: CH2=CHR¹ or formula R¹CH=CHR¹, wherein:
      R¹ is (CH₂)ᵣQ where Q = O(CH₂CH₂O)ₚH, OCOCH₃, OSO₃Y, SO₃Y, COOY, NHᵥZ_{w}, aryl, arylsulfonic acid or halide wherein x = 1-5; Y = H, Na, K or NH₄; p = 0-20, r = 0-4; Z = alkyl group, v = 0 or 1, w = 1 or 2 and when v = 0 then w = 2 and when v =1 then w = 2;
b. Removing any volatile by-products by metathesis; and
c. Isolating the surfactant and or surfactant intermediate from the metathesis catalyst.
The mixture or compositions can be incorporated into various consumer products such as shampoo and conditioners and various cleaning products forming new shampoo compositions and cleaning compositions. The mixture of surfactant composition of Process 1 can be incorporated into a cleaning composition such as a laundry detergent, shampoo, dishwashing detergent or hard surface cleaning composition.

### DETAILED DESCRIPTION OF THE INVENTION

1. A mixture comprising a first composition and a second composition, wherein said first composition is represented by the general formula:

   H[CH₂CH₀₊ₐ (CH₃)CH₁₊ₐ CH₂]ₓCH₂CH_{0+b} (CH_{1+b}R²)(CH_{1+c} CH_{1+c})R¹

   wherein x = 1-5; a = 1; b = 1; c = 0; R¹=(CH2)ᵣ-Q and R² = H, wherein:
   iv. Q = O(CH2CH2O)ₚH, OCOCH₃,
   v. p = 0; and
   vi. r = 1;
   and wherein said second composition is represented by the general formula:

   H[CH₂CH₀₊ₐ (CH₃)CH₁₊ₐ CH₂]ₓCH₂CH_{0+b} (CH_{1+b}R²)(CH_{1+c} CH_{1+c})R¹

   wherein x = 1-5; a = 1; b = 0; c = 1; R¹= H and R² = (CH2)ᵣQ wherein:
   iv. Q = O(CH2CH2O)ₚH, OCOCH₃,
   v. p = 0; and
   vi. r = 1

In one embodiment the composition has a bio-based content of greater than 50%. In another embodiment the composition has a bio-based content of essentially 100%. The polybranched mono-olefin or poly-olefin containing compositions are prepared and/or modified by the following process ("Process 1"):
a. Co-metathesizing an isoprenoid and a second compound with a metathesis catalyst neat or in a solvent, wherein
   i. the isoprenoid has a structure of formula:

      H[CH₂CH₀₊ₐ (CH₃)CH₁₊ₐCH₂]ₓCH₂CH_{0+b} (CH_{2+b})(CH_{1+c}CH_{2+c});

      and a = 0 or 1, b= 0 or 1, c= 0 or 1 and b+c = 0 or 1;
   ii. the second compound has structure of either formula: CH2=CHR¹ or formula R¹CH=CHR¹, wherein:
      R¹ is (CH₂)ᵣQ where Q = O(CH₂CH₂O)ₚH, OCOCH₃, OSO₃Y, SO₃Y, COOY, NHᵥZ_{w} , aryl, arylsulfonic acid or halide wherein x = 1-5; Y = H, Na, K or NH₄; p = 0-20, r = 0-4; Z = alkyl group, v = 0 or 1, w = 1 or 2 and when v = 0 then w = 2 and when v =1 then w = 2;
b. Removing any volatile by-products by metathesis; and
c. Isolating the surfactant and or surfactant intermediate from the metathesis catalyst.
The mixture of compositions can be incorporated into various consumer products such as shampoo and conditioners and various cleaning products forming new shampoo compositions and cleaning compositions. The mixture of surfactant composition of "Process 1" is incorporated into a cleaning composition such as a laundry detergent, shampoo, dishwashing detergent or hard surface cleaning composition.

### Definitions

As used herein, the following terms shall have the meaning specified thereafter:

"Farnesene" refers to a set of six closely related chemical compounds which all are sesquiterpenes. α-Farnesene and β-farnesene are isomers, differing by the location of one double bond. α-Farnesene (structure (b) above) is 3,7,11-trimethyl-1,3,6,10-dodecatetraene and β-farnesene (structure (a) above) is 7,11-dimethyl-3-methylene-1,6,10-dodecatriene. The alpha form can exist as four stereoisomers that differ about the geometry of two of its three internal double bonds (the stereoisomers of the third internal double bond are identical). The beta isomer exists as two stereoisomers about the geometry of its central double bond. Two of the α-farnesene stereoisomers are reported to occur in Nature. (E,E)-α-Farnesene is the most common isomer. It is found in the coating of apples, and other fruits. (Z,E)-α-Farnesene has been isolated from the oil of perilla. β-Farnesene has one naturally occurring isomer. The E isomer is a constituent of various essential oils. Several plants, including potato species, have been shown to synthesize this isomer.

"Bio-based content" refers to the amount of carbon from a renewable resource in a material as a percent of the mass of the total organic carbon in the material, as determined by ASTM D6866-10, method B. Note that any carbon from inorganic sources such as calcium carbonate is not included in determining the bio-based content of the material.

"Renewable" refers to a material that can be produced or is derivable from a natural source which is periodically (e.g., annually or perennially) replenished through the actions of plants of terrestrial, aquatic or oceanic ecosystems (e.g., agricultural crops, edible and non-edible grasses, forest products, seaweed, or algae), or microorganisms (e.g., bacteria, fungi, or yeast).

"Renewable resource" refers to a natural resource that can be replenished within a 100 year time frame. The resource may be replenished naturally, or via agricultural techniques. Renewable resources include plants, animals, fish, bacteria, fungi, and forestry products. They may be naturally occurring, hybrids, or genetically engineered organisms. Natural resources such as crude oil, coal, and peat which take longer than 100 years to form are not considered to be renewable resources.

"Sulfonate" refers to the anion (*i.e*., conjugate base) of a sulfonic acid. The pH of the product solution determines the relative amount of a sulfonate versus a sulfonic acid in a preparation according to the Henderson-Hasselbalch equation {pH=pKa + log([A⁻]/[HA]}, where pKa is -log(Ka). Ka is the acid dissociation constant of a sulfonic acid. For example, the pKa of *p*-toluenesulfonic acid is -2.8 in water. Thus, at or near neutral pH, *p*-toluenesulfonic acid will exist primarily as its sulfonate anion. As used herein, "sulfonic acid" and "sulfonate" are both meant to encompass the other.

### Poly-branched Poly-olefin Hydrophobe Structures are designated as compounds i. in the subsequent process detail

In one embodiment, the mixture of compositions of the present invention are derived from isoprenoid feedstocks. These can be described by the following formula:

H[CH₂CH₀₊ₐ (CH₃)CH₁₊ₐ CH₂]ₓCH₂CH_{0+b} (CH_{2+b})(CH_{1+c} CH_{2+c});

and a = 0 or 1, b= 0 or 1, c= 0 or 1 and b+c = 0 or 1, and for the present invention the feedstocks must provide a=1 and b= 0 or 1 and c= 0 or 1, to provide the mixtures claimed.

Another element is that certain poly-unsaturated feedstocks, either derived from the isoprenoids or prepared directly can provide the foundation of the hydrophobe of the new surfactant intermediates and subsequent surfactant compositions for use in consumer products. To better illustrate the possible complexity of the novel and preferred surfactant intermediates for the invention, structures (a) to (j) below are shown. These are only a few of hundreds of possible preferred structures that make up the potential isoprenoid feedstocks, and should not be taken as limiting the invention.

| | |
|---|---|
| 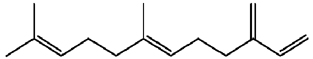 | 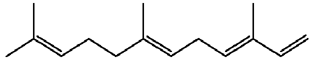 |
| (a) (*E*)-7,11-dimethyl-3-methylene-dodeca-1,6,10-triene | (b) (3*E*,6*E*)-3,7,11-trimethyldodeca-1,3,6,10-tetraene |
| COMMON NAME: Beta Farnesene | Common Name: Alpha Farnesene |
| 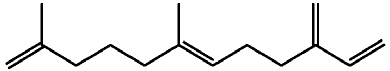 | 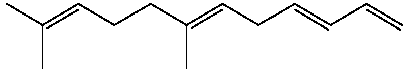 |
| (c) (*E*)-2,6-dimethyl-10-methylenedodeca-1,6,11-triene | (d) (3*E*,6*E*)-7,11-dimethyldodeca-1,3,6,10-tetraene |
| 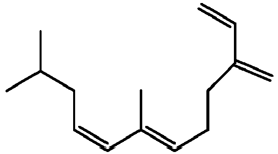 | 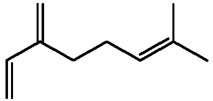 |
| (e) (6*E*,8*Z*)-7,11-dimethyl-3-methylenedodeca-1,6,8-triene | (f) 7-methyl-3-methyleneocta-1,6-diene COMMON NAME: Beta-Myrcene |
| 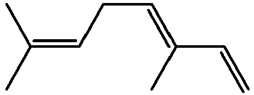 | 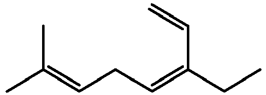 |
| (g) (*E*)-3,7-dimethylocta-1,3,6-triene | (h) (*Z*)-3-ethyl-7-methylocta-1,3,6-triene |
| 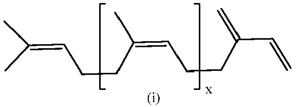 | 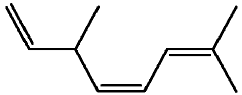 |
| | (j) (Z)-3,7-dimethy1octa-1,4,6-triene |

Compound (a), (b), (c) and (e) can be sourced from:
i. natural derived farnesene extracted from pre-existing plants and organisms;
ii. farnesene obtained via genetically modified organisms;
iii. synthetically derived trimers of isoprene;
iv. mixtures thereof.

Other examples of illustrated poly-branched poly-olefins are not sourced from i, ii, iii, or iv above. These examples are less preferred.

Some preferred poly-branched mono- or poly-olefins for conversion to the novel intermediates are illustrated by structures k, l, m and n.

| | |
|---|---|
| 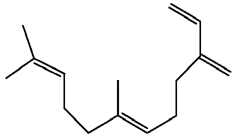 | 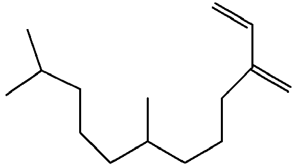 |
| (k) (E)-7,11-dimethyl-3-methylene-1,6,10-dodecaene | (l) 7,11-dimethyl-3-methylenedodec-1-ene |
| 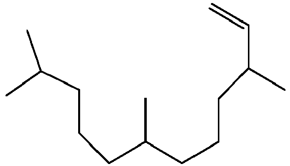 | 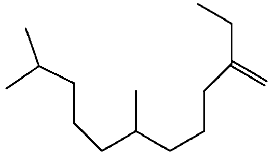 |
| (m) 3,7,11,-trimethyldodec-1-ene | (n) 2,6-dimethyl-10-methylenedodecane |

### i. Naturally derived farnesene extracted from pre-existing plants and organisms:

Examples of naturally derived farnesenes and potentially other structures illustrated can come from the class of natural materials called terpenes. Terpenes are a large and varied class of hydrocarbons, produced primarily by a wide variety of plants, particularly conifers and other pines, though also by some insects such as swallowtail butterflies. As many of these materials isolated from plants and other natural organisms often are present as gross mixtures, it may be desirable to purify the components before use in the processes of the invention. See U.S. 4,605,783.

### ii. Farnesene obtained via genetically modified organisms:

Several recent examples now allow for farnesene and other isoprene derivatives to be supplied via genetically modified organisms. Examples of such sources can be found in U.S. Patent 7,399,323 B2. This reference describes potential use of farnesane as fuel derived via genetically engineered farnesene. Another source of genetically engineered farnesene and isoprenes is disclosed in U.S. Patent 6,872,556 B2.

### iii. Synthetically derived trimers of isoprene:

Synthetically derived trimers can be obtained from various sources, two of which are shown in Patents JP 52031841 and JP 48040705. JP 48040705 teaches a process to make compound (b) as illustrated above. The process involves oligomerization of isoprene in the presence of divalent Ni, phosphine derivatives, and organomagnesium compounds to give high yields i.e. 75% of compound (b). Other synthetic processes to derive trimers are available.

Mixtures of any of the above disclosed non-limiting feedstocks can be used in the processes of the invention as well as isomeric forms.

### Surfactant Intermediates, Surfactants and Subsequent Surfactant Compositions

Previously described processes to make surfactant intermediates and surfactants based on acyclic isoprenoids are in the following published patents: (US8,299,308; US8,232,432 and US8,044,249). The processes, here employed, such as hydroformylation technology can require large scale plants to be cost effective for use in detergent applications. One of the advantages of the present invention is the ability to prepare the mixture of compositions including those disclosed in the above summary of the invention without the need for large scale manufacturing plants and thus have utility to be located in the geographical regions where the products are made without the expense of shipping to the various locations. This is made possible by the use of metathesis chemistry to link the various surfactant head groups to the acyclic isoprenoid feedstocks as described in detail for Process I below.

### Preparing surfactant intermediates, surfactants, and subsequent surfactant compositions ("Process 1"):

Process 1 comprises the following steps:
a. Co-metathesizing an isoprenoid and a second compound with a metathesis catalyst neat or in a solvent, wherein
   iii. the isoprenoid has a structure of formula:

      H[CH₂CH₀₊ₐ (CH₃)CH₁₊ₐ CH₂]ₓCH₂CH_{0+b} (CH_{2+b})(CH_{1+c} CH_{2+c});

      and a = 0 or 1, b= 0 or 1, c= 0 or 1 and b+c = 0 or 1;
   iv. the second compound has structure of either formula: CH2=CHR¹ or formula R¹CH=CHR¹, wherein:
      R¹ is (CH₂)ᵣQ where Q = O(CH₂CH₂O)ₚH, OCOCH₃, OSO₃Y, SO₃Y, COOY, NHᵥZ_{w}, aryl, arylsulfonic acid or halide wherein x = 1-5; Y = H, Na, K or NH₄; p = 0-20, r = 0-4; Z = alkyl group, v = 0 or 1, w = 1 or 2 and when v = 0 then w = 2 and when v =1 then w = 2;
b. Removing any volatile by-products by metathesis; and
c. Isolating the surfactant and or surfactant intermediate from the metathesis catalyst.

This process embodiment can be illustrated by the following PROCESS SCHEME I which uses, as a non-limiting example, beta farnesene as feedstock.

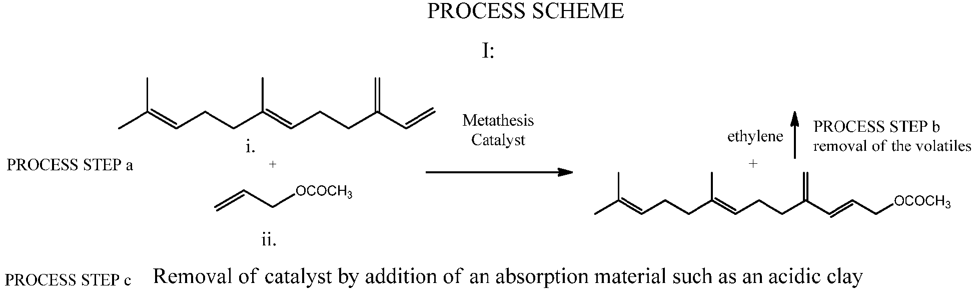

### CO-metathesis compounds for process step a:

The co-metathesis compounds used in process step b are CH2=CHR¹ or R¹CH=CHR¹, Wherein: R¹ is (CH₂)ᵣQ where Q = O(CH₂CH₂O)ₚH, OCOCH₃, OSO₃Y, SO₃Y, COOY, NHᵥZ_{w}, aryl, arylsulfonic acid or halide wherein x = 1-5; Y = H, Na, K or NH₄; p = 0-20, r = 0-4; Z = alkyl group; v = 0 or 1, w = 1 or 2 and when v = 0 then w = 2 and when v =1 then w = 2.

Non-limiting examples of suitable co-metathesis compounds are illustrated below.

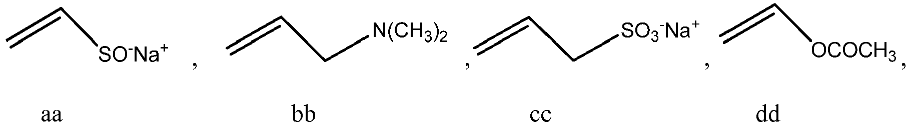

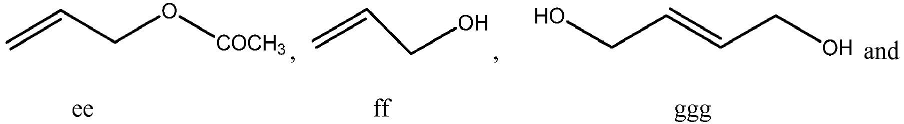

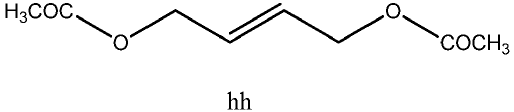

Preferred compounds are:

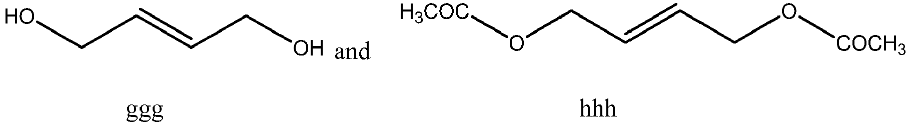

These materials preferred compounds can also be prepared from the corresponding precursors, allyl alcohol and allyl acetate by self metathesis or are generated in situ in some cases during co-metathesis with the acyclic isoprenoids.

Another aspect of the allyl OH and allyl acetate and the above preferred materials is they are now accessible via a renewable carbon process from glycerin in US 8,273,926. This allows for formation of essentially 100% renewable product of formula I. Thus, the compositions of the invention can have bio-based content of essentially 100% if used with a 100% bi-based content isoprenoid.

### Metathesis Catalysts for process step a

The catalyst used in the metathesis reaction step b can be any metathesis catalyst or catalyst system useful to catalyze the metathesis reaction of the invention to the desired extent. Any known or future metathesis catalyst can be employed alone, or in combination, with one or more additional catalysts. In some embodiments, the catalyst is quenched and distilled before use. Quenching can be carried out by methyl vinyl ether or removal of the catalyst by absorption onto, e.g., clays. Examples of suitable metathesis catalysts include metal carbene catalysts based on transition metals, such as, for example, ruthenium, chromium, rhenium, tungsten/tin, molybdenum, osmium, titanium, and mixtures thereof. Preferred metathesis catalysts can be based on transition metals selected from the group consisting of a ruthenium catalyst, a molybdenum catalyst, a tungsten/tin catalyst, a rhenium catalyst, a titanium catalyst, and mixtures thereof.

Nonlimiting, specific examples of catalysts appropriate for the production of the compositions of formula I include the Tebbe complex, a tungsten dicarbonyl complex (e.g., W(CO)₅CPhOCH₃, W(CO)₅CPh₂) Grubbs first generation catalyst [Ru(Cl)₂(PCy₃)₂CHPh], Grubbs second generation catalyst [Ru(Cl)₂(PCy₃)₂(NHC)CHPh], where NHC is a bulky *N*-heterocyclic carbene ligand H₂IMes, a Schrock carbene complex (e.g., Ta=CH-*t*-Bu(CH₂-*t*-Bu)₃, [W(O)(=CH-*t*-Bu)(PEt₃)₂Cl₂]), or any of the catalysts described in Vougioukalakis and Grubbs, Chem. Rev., 110(3): 1746-1787 (2010), and U.S. Patent Application Nos. 2009/0217568 and 2010/0145086, each incorporated herein by reference. Other examples of suitable catalysts include SASOL's Ru-alkylidene catalyst that contains a phosphorus containing ligand, such as phosphabicylononane, as described in U.S. Patent No. 7,671,224, U.S. Patent Application Publication No. 2008/0221345, and PCT Patent Application Publication No. 2007/010453, each incorporated herein by reference, examples of which are shown below.

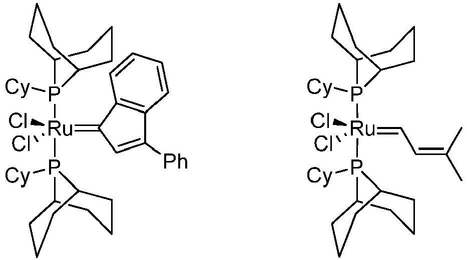

Hoveyda-Grubbs catalysts are also suitable catalysts for the invention, as described in Marvey et al., "Ruthenium Carbene Mediated Metathesis of Oleate-Type Fatty Compounds," Int. J. Mol. Sci. 9, 615-625 (2008), and WO 2010/062958, each incorporated herein by reference. An example of a Hoveyda-Grubbs catalyst is shown below. 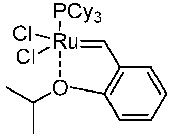 Polymer-bound catalysts, examples of which are described in Buchmeiser, "Polymer-Supported Well-Defined Metathesis Catalysts," Chem. Rev., 109, 303-321, 2009, incorporated herein by reference, also can be used for the metathesis reaction of the invention.
In some embodiments, the metathesis reaction is carried out in the presence of a phenolic compound (e.g., phenol, substituted phenol), as described in U.S. Patent Application Publication No. 2006/0211905. The phenolic compound enhances the turnover of the catalyst, which slows down deactivation of the catalyst.

### Metathesis Reaction Conditions for process step a

The metathesis reaction can be carried out neat or in an organic solvent. The presence of a solvent improves mixing and, if added to isoprenoid and or co-metathesis agent and partially distilled off before reaction, helps remove traces of water which can poison some metathesis catalysts (e.g., tungsten hexachloride). However, a solvent can be considered optional as well. The more commonly used solvents in metathesis reactions include aliphatic solvents (e.g., saturated hydrocarbons) and aromatic solvents (e.g., benzene, chlorobenzene, and toluene). The aliphatic solvents are preferred over the aromatic solvents because of a reduced tendency to interact with the reactants. In some preferred embodiments, the solvent is a saturated hydrocarbon that boils in the range of about 50°C to about 120°C (e.g., commercial hexane).

In some embodiments, the metathesis reaction is carried out at a temperature of about 20°C to about 260°C, preferably about 50°C to about 120°C. The reaction does not proceed to a noticeable degree at temperatures below about 20°C. The rate of the reaction increases with increasing temperature. Temperatures above about 260°C, however, are undesirable because the starting materials begin to degrade.

### Process Step b

Removal of volatile compounds and by-products are accomplished with mild vacuum or by separate higher vacuum post after metathesis reaction is complete by standard means.

### Process Step c

Removal of the catalyst was accomplished by standard treatment with a bleaching clay (BASF F-160®). If excess of the co-metathesis agent is used it can be removed by standard vacuum distillation. If necessary, final purification can be accomplished by chromatography (silica gel column). Any solvent used is removed by distillation under vacuum.

For embodiment where further processing of the product of process step c is needed the following processes can be used:

### Hydrogenation detail

Hydrogenation is carried out with a variety of catalysts ranging from Nickel on Kieselguhr Rhodium on Silica, Palladium on Kieselguhr are other examples of catalysts which can be used for the reduction of product of step d. Reaction conditions vary from 20°C to about 130°C, a hydrogen pressure ranging from 100 psig to about 2000 psig of hydrogen and catalyst loadings can typically be in range of from 1 to 5% on the substrate relative to product of step d. Reaction times will vary according to catalyst ratio, temperature chosen and hydrogen pressure. Typical conditions are 100°C at 1000 psig for 5-16 hours in batch mode. The process is not limited to batch reactions, but continuous reaction can also be applied to the invention.

### Further processing of the product of hydrogenation may include the following:

Ethoxylation, ethoxylation and sulfation and neutralization or sulfation and neutralization is well known to one skilled in the art. Most industrial ethoxylations are performed with base catalysts in batch mode at elevated temperatures. Sulfations, industrially, are typically done on falling film reactor systems using SO₃ as the sulfating agent. In the case where the functional group added by process step of metathesis is an amine quaternization via process step f is also considered well known by one skilled in the art using various quaternization agents such as dimethyl sulfate, methyl chloride, methyl bromide.

### Poly-branched Mono-unsaturated and Poly-unsaturated Functional Intermediates, Detergent Alcohols and Surfactants:

Illustrative poly-branched mono-unsaturated and poly-unsaturated intermediates, detergent alcohols and surfactants, are shown below.

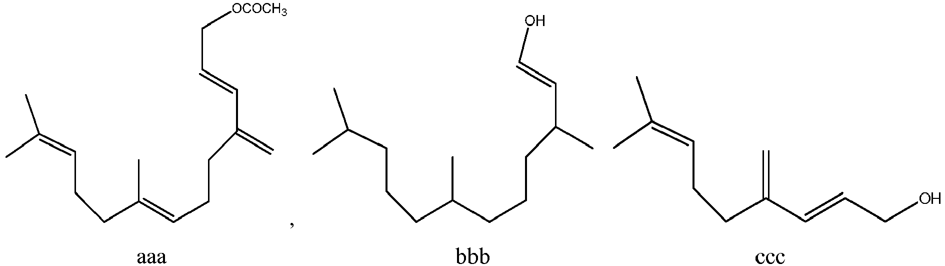

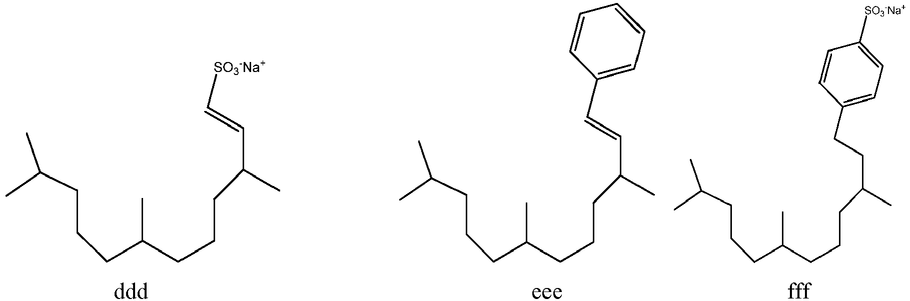

The compounds aaa-fff are non-limiting examples which can be used in shampoos, dishwashing and/or hard surface cleaners in some cases without further modification or converted by process I and other processes such as ethoxylation or ethoxylation and sulfation, or sulfation to various surfactant structures. Some further non-limiting examples of these intermediates and surfactants are shown below.

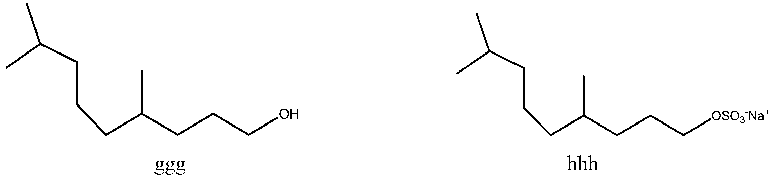

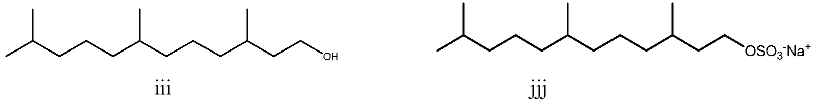

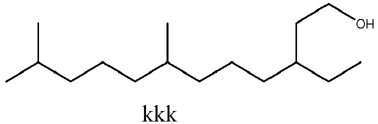

Some examples of products of Process I and further processing to surfactants are illustrated below by 111 through ppp :

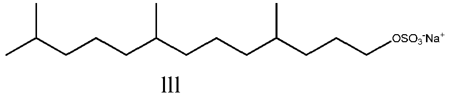

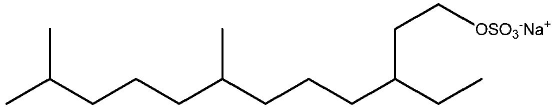

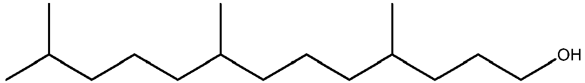

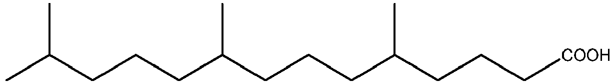

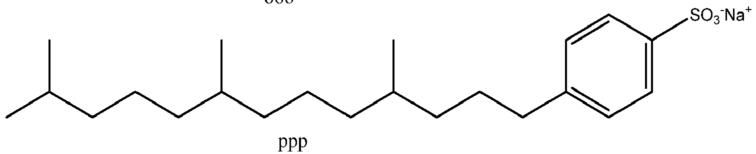

The compositions of the invention can have varying degrees of bio-based content. Bio-based content can be greater than 50%. In some cases the bio-based content can be essentially 100%.

### Bio-based content:

As used herein, the following terms shall have the meaning specified thereafter:

"Bio-based content" refers to the amount of carbon from a renewable resource in a material as a percent of the mass of the total organic carbon in the material, as determined by ASTM D6866-10, method B. Note that any carbon from inorganic sources such as calcium carbonate is not included in determining the bio-based content of the material.

Renewable" refers to a material that can be produced or is derivable from a natural source which is periodically (e.g., annually or perennially) replenished through the actions of plants of terrestrial, aquatic or oceanic ecosystems (e.g., agricultural crops, edible and non-edible grasses, forest products, seaweed, or algae), or microorganisms (e.g., bacteria, fungi, or yeast).

"Renewable resource" refers to a natural resource that can be replenished within a 100 year time frame. The resource may be replenished naturally, or via agricultural techniques. Renewable resources include plants, animals, fish, bacteria, fungi, and forestry products. They may be naturally occurring, hybrids, or genetically engineered organisms. Natural resources such as crude oil, coal, and peat which take longer than 100 years to form are not considered to be renewable resources.

Assessment of the renewably based carbon in a material can be performed through standard test methods. Using radiocarbon and isotope ratio mass spectrometry analysis, the bio-based content of materials can be determined. ASTM International, formally known as the American Society for Testing and Materials, has established a standard method for assessing the bio-based content of materials. The ASTM method is designated ASTM D6866-10.

The application of ASTM D6866-10 to derive a "bio-based content" is built on the same concepts as radiocarbon dating, but without use of the age equations. The analysis is performed by deriving a ratio of the amount of organic radiocarbon (¹⁴C) in an unknown sample to that of a modern reference standard. The ratio is reported as a percentage with the units "pMC" (percent modern carbon).

The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.

"Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. It's gradually decreased over time with today's value being near 107.5 pMC. This means that a fresh biomass material such as corn could give a radiocarbon signature near 107.5 pMC.

Combining fossil carbon with present day carbon into a material will result in a dilution of the present day pMC content. By presuming 107.5 pMC represents present day biomass materials and 0 pMC represents petroleum derivatives, the measured pMC value for that material will reflect the proportions of the two component types. A material derived 100% from present day soybeans would give a radiocarbon signature near 107.5 pMC. If that material was diluted with 50% petroleum derivatives, for example, it would give a radiocarbon signature near 54 pMC (assuming the petroleum derivatives have the same percentage of carbon as the soybeans).

A biomass content result is derived by assigning 100% equal to 107.5 pMC and 0% equal to 0 pMC. In this regard, a sample measuring 99 pMC will give an equivalent bio-based content value of 92%.

Assessment of the materials described herein was done in accordance with ASTM D6866. The mean values quoted in this report encompasses an absolute range of 6% (plus and minus 3% on either side of the bio-based content value) to account for variations in end-component radiocarbon signatures. It is presumed that all materials are present day or fossil in origin and that the desired result is the amount of bio-based component "present" in the material, not the amount of bio-based material "used" in the manufacturing process.

### Poly-branched Surfactants

Surfactant compositions may be derived from the mixtures of poly-branched mono-unsaturated and poly-unsaturated detergent alcohols. The surfactants may be formed by way of any alcohol-to-surfactant derivatization process known in the industry.

Poly-branched mono-unsaturated and poly-unsaturated detergent alcohols may be converted into other useful polybranched surfactants such as cationic surfactants, zwitterionic surfactants, amine oxide surfactants, alkylpolyglycoside surfactants, soaps, fatty acids, and di alkyl cationic surfactants. Synthetic procedures for obtaining these materials from the parent polybranched alcohols may be found in the Kirk Othmer Encyclopedia of Chemical Technology or other documents in the chemical art.

The aforementioned cationic surfactants, zwitterionic surfactants, amine oxide surfactants, soaps, fatty acids, may also be combined with nonionic (AE) and anionic (AS, AES) surfactants derived from the aforementioned polybranched alcohols. These AE, AS, and AES materials are described in US8,299,308; US8,232,432 and US8,044,249 and involve treatment of the aforementioned polybranched alcohols with ethylene oxide optionally followed by sulfation.

### Polybranched cationic surfactants, amine oxide surfactants, and betaine surfactants.

Cationic surfactants may be derived from the abovementioned detergent alcohols. The aforementioned polybranched alcohols may be converted into tertiary amines via direct amination via reaction with secondary amines such as mono-ethanol amine (to provide the polybranched methyl, hydroxyethyl tertiary amine) or dimethyl amine (to provide the polybranched dimethyl tertiary amine). These processes are via direct amination in the presence of the amine at 230°C at atmospheric pressure (0.1-0.5 MPa) using copper chromite catalysts (from the polybranched alcohol) or noble metal, copper chelate, or copper carboxylate catalysts (from the polybranched aldehydes). The resulting polybranched tertiary amines are then converted to the hydroxyalkyl quat or trimethyl quats via reaction with methyl chloride or dimethyl sulfate.

Alternatively, to prepare the following amine oxides, the aforementioned polybranched tertiary amine is oxidized with hydrogen peroxide in water with bicarbonate buffer.

Alternatively, to prepare the following zwitterionic betaine surfactants, the aforementioned polybranched tertiary amine is reacted with 1,3-propane sultone, typically in acetone.

The amine oxides are highly desirable for their grease cleaning and foaming ability, and these properties are enhanced by the branching of the mixture of composition.

### Polybranched zwitterionic surfactants

The betaine classes of surfactants are also useful in enhancing the performance of the primary, mainframe surfactant, and having them derived from natural farnesene or isoprenoid sources provides additional sustainability benefits as well as enhanced cold water performance, and ease of preparing formulations.

### Polybranched Fatty acids and Soaps

Soaps and fatty acids are sometimes used in laundry detergents as adjunct surfactants, or as additives to provide mildness and other sensorial benefits. When soaps and fatty acids contain the aforementioned polybranched moieties, they gain important advantages in solubility. The following polybranched fatty acids and soaps are prepared via oxidation of the aforementioned polybranched alcohols via any of a number of oxidizing agents such as potassium permangenate, Jones reagent, or other techniques known in the art. (X = Na or other counterion, or hydrogen.)

### Polybranched dialkyl cationic surfactants (fabric softener actives)

Fabric softener actives may be derived from the abovementioned polybranched alcohols, and have advantages in phase stability and compaction, as well as excellent fabric softening. The di(polybranched-alkyl)-dimethyl quats may be prepared via direct amination of the aforementioned polybranched alcohols via reaction at high temperature with methyl amine [using copper chromite catalysts (from the polybranched alcohol) or noble metal, copper chelate, or copper carboxylate catalysts (from the polybranched aldehydes)], followed by quaternization with methyl chloride or dimethyl sulfate. The di(polybranched) diester quats are prepared by oxidation of the aforementioned polybranched alcohols or aldehydes with any of a number of oxidizing agents such as potassium permangenate, Jones reagent, or other techniques known in the art, followed by diesterification of N-methyldiethanolamine with the resulting polybranched carboxylic acids, followed by quaternization with methyl chloride or dimenthyl-sulfate.

### Surfactant Compositions and Products

The poly-branched surfactant composition comprising one or more derivatives of the detergent alcohol selected from the anionic, nonionic, cationic, amine oxide, and or zwitterionic mixtures thereof are outstandingly suitable as soil detachment and suspending promoting additives for laundry and other cleaning compositions. The dialkyl or diester quats are particularly well suited for fabric softener compositions.

The poly-branched surfactant compositions according to the present invention can be added to the laundry detergents, cleaning compositions, and fabric softener compositions in amounts of generally from 0.05 to 70% by weight, preferably from 0.1 to 40% by weight and more preferably from 0.25 to 10% by weight, based on the particular overall composition.

In addition, the laundry detergents and cleaning compositions generally comprise surfactants and, if appropriate, other polymers as washing substances, builders and further customary ingredients, for example cobuilders, cleaning polymers (modified and unmodified polycarboxylates, ethoxylated amines and derivatives thereof), complexing agents, bleaches, standardizers, graying inhibitors, dye transfer inhibitors, enzymes and perfumes.

The novel surfactant compositions of the present invention may be utilized in laundry detergents or cleaning compositions comprising a surfactant system comprising C₁₀-C₁₅ alkyl benzene sulfonates (LAS) and one or more co-surfactants selected from nonionic, cationic, anionic or mixtures thereof. The selection of co-surfactant may be dependent upon the desired benefit. In one embodiment, the co-surfactant is selected as a nonionic surfactant, preferably C₁₂-C₁₈ alkyl ethoxylates. In another embodiment, the co-surfactant is selected as an anionic surfactant, preferably C₁₀-C₁₈ alkyl alkoxy sulfates (AEₓS) wherein x is from 1-30. In another embodiment the co-surfactant is selected as a cationic surfactant, preferably dimethyl hydroxyethyl lauryl ammonium chloride. If the surfactant system comprises C₁₀-C₁₅ alkyl benzene sulfonates (LAS), the LAS is used at levels ranging from about 9% to about 25%, or from about 13% to about 25%, or from about 15% to about 23% by weight of the composition.

The surfactant system may comprise from 0% to about 7%, or from about 0.1% to about 5%, or from about 1% to about 4% by weight of the composition of a co-surfactant selected from a nonionic co-surfactant, cationic co-surfactant, anionic co-surfactant and any mixture thereof.

Non-limiting examples of nonionic co-surfactants include: C₁₂-C₁₈ alkyl ethoxylates, such as, NEODOL® nonionic surfactants from Shell; C₆-C₁₂ alkyl phenol alkoxylates wherein the alkoxylate units are a mixture of ethyleneoxy and propyleneoxy units; C₁₂-C₁₈ alcohol and C₆-C₁₂ alkyl phenol condensates with ethylene oxide/propylene oxide block alkyl polyamine ethoxylates such as PLURONIC® from BASF; C₁₄-C₂₂ mid-chain branched alcohols, BA, as discussed in US 6,150,322; C₁₄-C₂₂ mid-chain branched alkyl alkoxylates, BAEₓ, wherein x is from 1-30, as discussed in US 6,153,577, US 6,020,303 and US 6,093,856; alkylpolysaccharides as discussed in U.S. 4,565,647 Llenado, issued January 26, 1986; specifically alkylpolyglycosides as discussed in US 4,483,780 and US 4,483,779; polyhydroxy detergent acid amides as discussed in US 5,332,528; and ether capped poly(oxyalkylated) alcohol surfactants as discussed in US 6,482,994 and WO 01/42408.

Non-limiting examples of semi-polar nonionic co-surfactants include: water-soluble amine oxides containing one alkyl moiety of from about 10 to about 18 carbon atoms and 2 moieties selected from the group consisting of alkyl moieties and hydroxyalkyl moieties containing from about 1 to about 3 carbon atoms; water-soluble phosphine oxides containing one alkyl moiety of from about 10 to about 18 carbon atoms and 2 moieties selected from the group consisting of alkyl moieties and hydroxyalkyl moieties containing from about 1 to about 3 carbon atoms; and water-soluble sulfoxides containing one alkyl moiety of from about 10 to about 18 carbon atoms and a moiety selected from the group consisting of alkyl moieties and hydroxyalkyl moieties of from about 1 to about 3 carbon atoms(See WO 01/32816, US 4,681,704, and US 4,133,779).

Non-limiting examples of cationic co-surfactants include: the quaternary ammonium surfactants, which can have up to 26 carbon atoms include: alkoxylate quaternary ammonium (AQA) surfactants as discussed in US 6,136,769; dimethyl hydroxyethyl quaternary ammonium as discussed in 6,004,922; dimethyl hydroxyethyl lauryl ammonium chloride; polyamine cationic surfactants as discussed in WO 98/35002, WO 98/35003, WO 98/35004, WO 98/35005, and WO 98/35006; cationic ester surfactants as discussed in US Patents Nos. 4,228,042, 4,239,660 4,260,529 and US 6,022,844; and amino surfactants as discussed in US 6,221,825 and WO 00/47708, specifically amido propyldimethyl amine (APA).

Nonlimiting examples of anionic co-surfactants useful herein include: C₁₀-C₂₀ primary, branched chain and random alkyl sulfates (AS); C₁₀-C₁₈ secondary (2,3) alkyl sulfates; C₁₀-C₁₈ alkyl alkoxy sulfates (AEₓS) wherein x is from 1-30; C₁₀-C₁₈ alkyl alkoxy carboxylates comprising 1-5 ethoxy units; mid-chain branched alkyl sulfates as discussed in US 6,020,303 and US 6,060,443; mid-chain branched alkyl alkoxy sulfates as discussed in US 6,008,181 and US 6,020,303; modified alkylbenzene sulfonate (MLAS) as discussed in WO 99/05243, WO 99/05242 and WO 99/05244; methyl ester sulfonate (MES); and alphaolefin sulfonate (AOS).

The present invention also relates to a surfactant composition comprising C₈-C₁₈ linear alkyl sulfonate surfactant and a co-surfactant. The compositions can be in any form, namely, in the form of a liquid; a solid such as a powder, granules, agglomerate, paste, tablet, pouches, bar, gel; an emulsion; types delivered in dual-compartment containers; a spray or foam detergent; premoistened wipes (i.e., the cleaning composition in combination with a nonwoven material such as that discussed in US 6,121,165, Mackey, et al.); dry wipes (i.e., the cleaning composition in combination with a nonwoven materials, such as that discussed in US 5,980,931, Fowler, et al.) activated with water by a consumer; and other homogeneous or multiphase consumer cleaning product forms.

In another embodiment, the cleaning composition of the present invention is a liquid or solid laundry detergent composition. In another embodiment, the cleaning composition of the present invention is a hard surface cleaning composition, preferably wherein the hard surface cleaning composition impregnates a nonwoven substrate. As used herein "impregnate" means that the hard surface cleaning composition is placed in contact with a nonwoven substrate such that at least a portion of the nonwoven substrate is penetrated by the hard surface cleaning composition, preferably the hard surface cleaning composition saturates the nonwoven substrate. The cleaning composition may also be utilized in car care compositions, for cleaning various surfaces such as hard wood, tile, ceramic, plastic, leather, metal, glass. This cleaning composition could be also designed to be used in a personal care and pet care compositions such as shampoo composition, body wash, liquid or solid soap and other cleaning composition in which surfactant comes into contact with free hardness and in all compositions that require hardness tolerant surfactant system, such as oil drilling compositions.

In another embodiment the cleaning composition is a dish cleaning composition, such as liquid hand dishwashing compositions, solid automatic dishwashing compositions, liquid automatic dishwashing compositions, and tab/unit does forms of automatic dishwashing compositions.

Quite typically, cleaning compositions herein such as laundry detergents, laundry detergent additives, hard surface cleaners, synthetic and soap-based laundry bars, fabric softeners and fabric treatment liquids, solids and treatment articles of all kinds will require several adjuncts, though certain simply formulated products, such as bleach additives, may require only, for example, an oxygen bleaching agent and a surfactant as described herein. A comprehensive list of suitable laundry or cleaning adjunct materials can be found in WO 99/05242.

Common cleaning adjuncts include builders, enzymes, polymers not discussed above, bleaches, bleach activators, catalytic materials and the like excluding any materials already defined hereinabove. Other cleaning adjuncts herein can include suds boosters, suds suppressors (antifoams) and the like, diverse active ingredients or specialized materials such as dispersant polymers (e.g., from BASF Corp. or Rohm & Haas) other than those described above, color speckles, silvercare, anti-tarnish and/or anticorrosion agents, dyes, fillers, germicides, alkalinity sources, hydrotropes, anti-oxidants, enzyme stabilizing agents, pro-perfumes, perfumes, solubilizing agents, carriers, processing aids, pigments, and, for liquid formulations, solvents, chelating agents, dye transfer inhibiting agents, dispersants, brighteners, suds suppressors, dyes, structure elasticizing agents, fabric softeners, anti-abrasion agents, hydrotropes, processing aids, and other fabric care agents, surface and skin care agents. Suitable examples of such other cleaning adjuncts and levels of use are found in U.S. Patent Nos. 5,576,282, 6,306,812 B1 and 6,326,348 B1.

### Method of Use

The mixture of compositions can be used in a method for cleaning a targeted surface. As used herein "targeted surface" may include such surfaces such as fabric, dishes, glasses, and other cooking surfaces, hard surfaces, hair or skin. As used herein "hard surface" includes hard surfaces being found in a typical home such as hard wood, tile, ceramic, plastic, leather, metal, glass. Such method includes the steps of contacting the mixture of compositions comprising the modified polyol compound, in neat form or diluted in wash liquor, with at least a portion of a targeted surface then optionally rinsing the targeted surface. Preferably the targeted surface is subjected to a washing step prior to the aforementioned optional rinsing step. For purposes of the present invention, washing includes, but is not limited to, scrubbing, wiping and mechanical agitation.

As will be appreciated by one skilled in the art, the cleaning compositions are ideally suited for use in home care (hard surface cleaning compositions) and/or laundry applications.

The composition solution pH is chosen to be the most complimentary to a target surface to be cleaned spanning broad range of pH, from about 5 to about 11. For personal care such as skin and hair cleaning pH of such composition preferably has a pH from about 5 to about 8 for laundry cleaning compositions pH of from about 8 to about 10. The compositions are preferably employed at concentrations of from about 150 ppm to about 10,000 ppm in solution. The water temperatures preferably range from about 5 °C to about 100 °C.

For use in laundry cleaning compositions, the compositions are preferably employed at concentrations from about 150 ppm to about 10000 ppm in solution (or wash liquor). The water temperatures preferably range from about 5°C to about 60°C. The water to fabric ratio is preferably from about 1:1 to about 20:1.

The method may include the step of contacting a nonwoven substrate impregnated with an embodiment of the composition of the present invention As used herein "nonwoven substrate" can comprise any conventionally fashioned nonwoven sheet or web having suitable basis weight, caliper (thickness), absorbency and strength characteristics. Examples of suitable commercially available nonwoven substrates include those marketed under the tradename SONTARA® by DuPont and POLYWEB® by James River Corp.

As will be appreciated by one skilled in the art, the cleaning compositions are ideally suited for use in liquid dish cleaning compositions. The method for using a liquid dish composition comprises the steps of contacting soiled dishes with an effective amount, typically from about 0.5 ml. to about 20 ml. (per 25 dishes being treated) of the liquid dish cleaning composition diluted in water.

### EXAMPLES

### Example 1 - Preparation of 8,12-dimethyl-4-methylenetrideca-2,7,11-trien-1-yl acetate (Process I Steps a,b and c)

1,2-dichloroethane, allyl acetate, and beta farnesene were stored over molecular sieves and degassed with argon before use. Allyl acetate (50 ml, 0.093 mol) and beta farnesene (22.3ml, 0.46 mol) were added to a 250 ml round bottom 3 neck flask fitted with a thermocouple, condenser, stir bar, and rubber septum, then was stirred and heated to 45°C. Once heated, Hoveyda-Grubbs 2^{nd} Gen Cat (0.58 g, 0.93 mmol) which was dissolved in 1,2-dichloroethane (5 ml) was slowly added to the farnesene/ allyl acetate solution and a sweep of argon was placed on the reaction mixture for 10 minutes. This was allowed to react 16 hr. The reaction mixture was cooled to room temp and the reaction mixture was treated 2 X 5.0 grams with bleaching clay (BASF F-160®). Unreacted ally acetate was removed by vacuum distillation and resulting product was purified by chromatography (silica gel column) to yield 8,12-dimethyl-4-methylenetrideca-2,7,11-trien-1-yl acetate (0.51 g) as an oil. MS (EI) *m*/*z* 276.1 (M)⁺. The 8,12-dimethyl-4-methylenetrideca-2,7,11-trien-1-yl acetate is represented by the following formula:

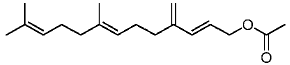

### Example 2 - Preparation of 8,12-dimethyl-4-methylenetrideca-2,7,11-trien-1-ol

Potassium carbonate (0.33 g, 0.23 mol) was dissolved in 5 ml of water. This was added to a solution of 8,12-dimethyl-4-methylenetrideca-2,7,11-trien-1-yl acetate (0.51 g,) in MeOH (10 ml). Once added, MeOH, H2O, and THF was added to make a clear solution. This was stirred for 1 hr and the organics were removed in vacuo. To the remaining aqueous layer was added 10 ml of brine and this was extracted 2X 50 ml Et₂O and 1 X 50 ml EtOAc. The organic layers were combined, washed with brine, and dried over Na₂SO₄. Solvent was removed in vacuo to yield a crude product which was purified by column chromatography to yield 8,12-dimethyl-4-methylenetrideca-2,7,11-trien-1-ol as an oil (30 mg). ¹H NMR (300 MHz, CHLOROFORM-*d*) δ ppm 1.61 (s, 6 H) 1.69 (d, *J*=0.94 Hz, 3 H) 1.89 - 2.35 (m, 8 H) 4.24 (dd, *J*=5.85, 1.32 Hz, 2 H) 5.02 (d, *J*=8.31 Hz, 2 H) 5.06 - 5.21 (m, 2 H) 5.89 (m, 1H) 6.29 (d, *J*=15.86 Hz, 1H); ¹³CNMR (75 MHz, CHLOROFORM-*d*) δ ppm 16.05, 17.67, 25.67, 26.59, 26.69, 32.10, 39.68, 63.75, 115.83, 123.86, 124.30, 127.58, 131.31, 133.56, 135.46, 145.16; MS (EI) *m*/*z* 234.2 (M)⁺; purity by GC 95.4%. The 8,12-dimethyl-4-methylenetrideca-2,7,11-trien-1-ol is represented by the following formula:

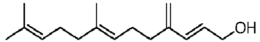

### Example 3 - Preparation of 4,8,12-trimethyltridec-2-en-1-yl acetate

The following reaction was performed in oven dried glassware. 1,2-dichloroethane, cis-1,4-diacetoxy-2-butene, and 3,7,11-trimethyldodec-1-ene were stored over 4A molecular sieves, degassed with argon before use and reaction was performed under argon. Cis-1,4-diacetoxy-2-butene (19.6 ml, 0.135 mol) and 3,7,11-trimethyldodec-1-ene (11.4 g, 0.054 mol) were added to a ml 250 ml round bottom flask fitted with a stir bar, thermocouple, condenser, and rubber septum. Next, 1,2-dichloroethane (20 ml) was added and this was stirred and heated to 60°C. Once heated, Hoveyda-Grubbs 2^{nd} Gen Cat (340 mg, 0.54 mmol) which was dissolved in 1,2-dichloroethane (5 ml) was slowly added to the reaction mixture. The reaction mixture was stirred for 1 hour then cooled to room temp and catalyst was removed by treatment with BASF F-160 bleaching clay (2 x 4 g). Solvent was removed in vacuo to yield the crude product which was purified by vacuum distillation to yield 4,8,12-trimethyltridec-2-en-1-yl acetate as a clear oil (8.6 grams). ¹H NMR (600 MHz, CHLOROFORM-*d*) δ ppm 0.73 - 1.42 (m, 25 H) 1.44 - 1.63 (m, 1H) 1.95 - 2.25 (m, 4 H) 4.35 - 4.74 (m, 2 H) 5.35 - 5.56 (m, 1H) 5.57 - 5.76 (m, 1H); ¹³C NMR (151 MHz, CHLOROFORM-*d*) δ ppm 20.06, 20.08, 20.48, 20.58, 21.43, 23.02, 23.12, 25.01, 25.02, 25.21, 28.38, 33.11, 36.78, 37.33, 37.48, 37.49, 37.68, 37.73, 39.75, 65.81, 122.30, 122.35, 142.69, 142.73, 171.21; MS (EI) *m*/*z* 222.3 (M-60)⁺; purity by GC 91.6%. The 4,8,12-trimethyltridec-2-en-1-yl acetate is represented by the following formula:

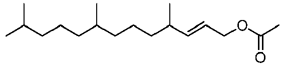

### Example 4 - Preparation of 4,8,12-trimethyltridecyl acetate

5% palladium on carbon (0.225 grams) was added to a 60 ml pressure reactor followed by 4,8,12-trimethyltridec-2-en-1-yl acetate (2.9 grams, 0.010 mol), and i-PrOH (25 ml). This was then purged 3 X 5 bar with N₂ followed by 3 x 10 bar of H₂ and then a final charge 12 bar of H₂. The reaction mixture was stirred at 600 RPM and 25°C for 60 min. The reaction mixture was vented and purged 3 X 10 bar with N₂. The catalyst was filtered off and solvent was removed in vacuo to yield crude product which was purified by chromatography (SGC, 4% EtOAc in hexane) to yield 4,8,12-trimethyltridecyl acetate as a clear colorless oil (1.05 g). ¹H NMR (600 MHz, CHLOROFORM-*d*) δ ppm 0.71 - 1.02 (m, 12 H) 1.03 - 1.82 (m, 19 H) 2.05 (s, 3 H) 3.90 - 4.26 (m, 2 H); ¹³C NMR (151 MHz, CHLOROFORM-*d*) δ ppm 19.50, 19.56, 19.64, 19.71, 21.00, 22.60, 22.70, 24.37, 24.39, 24.77, 24.79, 26.14, 26.15, 27.95, 32.45, 32.47, 32.73, 32.76, 32.98, 33.08, 37.15, 37.20, 37.24, 37.31, 37.34, 37.36, 39.33, 64.96, 171.20; MS (EI) *m*/*z* 224.3 (M-60)⁺; purity by GC 100%. The 4,8,12-trimethyltridecyl acetate is represented by the following formula:

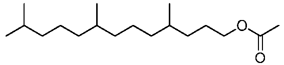

### Example 5 - Preparation of 4,8,12-trimethyltridecan-1-ol

Potassium carbonate 1.5 hydrate (0.42 g, 2.5 mmol) was dissolved in 5 ml of water. This was added to a solution of 4,8,12-trimethyltridecyl acetate (0.54 g, 1.8 mmol) in MeOH (10 ml). Once added, THF was added to make a clear solution. This was stirred for 2 hr and the organics were removed in vacuo. The remaining aqueous layer was extracted with 3 X 40 ml of EtOAc. The organic layers were combined, washed with brine, and dried over MgSO4. Solvent was removed in vacuo to yield 4,8,12-trimethyltridecan-1-ol as a clear oil (0.45 g) ¹H NMR (600 MHz, CHLOROFORM-*d*) δ ppm 0.79 - 0.98 (m, 12 H) 1.02 - 1.73 (m, 20 H) 3.64 (t, *J*=6.61 Hz, 2 H); ¹³C NMR (151 MHz, CHLOROFORM-*d*) δ ppm 19.58, 19.65, 19.66, 19.73, 22.61, 22.71, 24.41, 24.42, 24.78, 24.80, 27.96, 30.26, 30.27, 32.61, 32.63, 32.75, 32.77, 32.88, 32.98, 37.26, 37.30, 37.36, 37.40, 39.34, 63.46; MS (EI) 224.3 (M-18)⁺; purity by GC 96.7%. The 4,8,12-trimethyltridecan-1-ol is represented by the following formula:

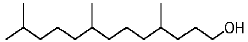

### Example 6 - Preparation of 4,8,12-trimethyltridec-2-en-1-ol

Potassium carbonate 1.5 hydrate (0.44 g, 2.6 mmol) was dissolved in 5 ml of water. This was added to a solution of 4,8,12-trimethyltridec-2-en-1-yl acetate (0.51 g, 1.8 mmol) in MeOH (10 ml). Once added, THF was added to make a clear solution. This was stirred for 2 hr and the organics were removed in vacuo. The remaining aqueous layer was extracted with 3 X 40 ml of EtOAc. The organic layers were combined, washed with brine, and dried over MgSO4. Solvent was removed in vacuo to yield 4,8,12-trimethyltridec-2-en-1-ol as a clear oil (0.42 g).
¹³C NMR (151 MHz, CHLOROFORM-*d*) δ ppm 19.67, 19.69, 20.31, 20.42, 22.61, 22.70, 24.65, 24.67, 24.78, 27.96, 32.70, 32.72, 36.28, 36.30, 37.11, 37.28, 37.32, 39.33, 63.91, 126.91, 126.95, 139.33, 139.38; MS (EI) 222.3 (M-18)⁺; purity by GC 89.9%. The 4,8,12-trimethyltridec-2-en-1-ol is represented by the following formula:

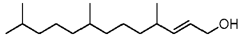

### Example 7 - Preparation of the ethoxylate of 4,8,12-trimethyltridec-2-en-1-ol containing 10 moles of ethylene oxide

10 moles of ethylene oxide is added to the 4,8,12-trimethyltridec-2-en-1-ol of example 6 by processes known to one skilled in the art to provide a nonionic surfactant. 22

### Example 8 - Preparation of the sulfate of 4,8,12-trimethyltridecan-1-ol

The 4,8,12-trimethyltridecan-1-ol produced in example 5 is sulfated by standard addition of SO₃ in a falling film reactor, as known in the art.

Unless otherwise noted, all component or composition levels are in reference to the active level of that component or composition, and are exclusive of impurities, for example, residual solvents or by-products, which may be present in commercially available sources.

All percentages and ratios are calculated by weight unless otherwise indicated. All percentages and ratios are calculated based on the total composition unless otherwise indicated.

## Claims

1. A mixture comprising a first composition and a second composition, wherein said first composition is represented by the general formula:
H[CH₂CH₀₊ₐ (CH₃)CH₁₊ₐ CH₂]ₓCH₂CH_{0+b} (CH_{1+b} R²)(CH_{1+c} CH_{1+c})R¹
wherein x = 1-5; a = 1; b = 1; c = 0; R¹=(CH2)ᵣ-Q and R² = H, wherein:
i. Q = O(CH2CH2O)ₚH, OCOCH₃,
ii. p = 0; and
iii. r = 1;
and wherein said second composition is represented by the general formula: H
[CH₂CH₀₊ₐ (CH₃)CH₁₊ₐ CH₂]ₓCH₂CH_{0+b} (CH_{1+b} R²)(CH_{1+c}CH_{1+c})R¹
wherein x = 1-5; a = 1; b = 0; c = 1; R¹= H and R² = (CH₂)ᵣQ wherein:
i. Q = O(CH2CH2O)ₚH, OCOCH₃,
ii. p = 0; and r = 1.

2. The composition of claim 1, wherein the bio-based content is greater than 50%.

## Patentansprüche

1. Mischung, eine erste Zusammensetzung und eine zweite Zusammensetzung umfassend, wobei die erste Zusammensetzung durch die allgemeine Formel dargestellt ist:
H[CH₂CH₀₊ₐ (CH₃)CH ₁₊ₐ CH₂]ₓCH₂CH_{0+b} (CH_{1+b} R²-)(CH_{1+c} CH_{1+c})R¹,
worin x = 1-5; a = 1; b = 1; c = 0; R¹=(CH2)ᵣ-Q und R² = H, worin:
i. Q = O(CH2CH2O)ₚH, OCOCH₃,
ii. p = 0; und
iii. r = 1;
und wobei die zweite Zusammensetzung durch die allgemeine Formel dargestellt ist:
H[CH₂CH₀₊ₐ (CH₃)CH₁₊ₐ CH₂]ₓCH₂CH_{0+b} (CH_{1+b} R²)(CH_{1+c} CH_{1+c})R¹,
worin x = 1-5; a = 1; b = 0; c = 1; R¹= H und R² = (CH2)ᵣQ, worin:
i. Q = O(CH2CH2O)ₚH, OCOCH₃,
ii. p = 0; und
r = 1;

2. Zusammensetzung nach Anspruch 1, wobei der bio-basierte Gehalt größer als 50 % ist.

## Revendications

1. Mélange comprenant une première composition et une deuxième composition, dans lequel ladite première composition est représentée par la formule générale :
H[CH₂CH₀₊ₐ (CH₃)CH₁₊ₐ CH₂]ₓCH₂CH_{0+b} (CH_{1+b} R²)(CH_{1+c} CH_{1+c})R¹
dans lequel x = 1 à 5 ; a = 1 ; b = 1 ; c = 0 ; R¹=(CH2)ᵣ-Q et R² = H, où :
i. Q = O(CH2CH2O)ₚH, OCOCH₃,
ii. p = 0 ; et
iii. r = 1 ;
et dans lequel ladite deuxième composition est représentée par la formule générale :
H[CH₂CH₀₊ₐ (CH₃)CH₁₊ₐ CH₂]ₓCH₂CH_{0+b} (CH_{1+b} R²)(CH_{1+c}CH_{1+c})R¹
dans lequel x = 1 à 5 ; a = 1 ; b = 0 ; c = 1 ; R¹= H et R² = (CH2)ᵣQ dans lequel :
i. Q = O(CH2CH2O)ₚH, OCOCH₃,
ii. p = 0 ; et
r= 1 ;

2. Composition selon la revendication 1, dans laquelle la teneur d'origine biologique est supérieure à 50 %.
